(19) Europäisches Patentamt
European Patent Office
Office européen des brevets

(11) Publication number: **0 040 104**
**B1**

(12) # EUROPEAN PATENT SPECIFICATION

(45) Date of publication of patent specification: **16.10.85**

(51) Int. Cl.4: **C 01 B 33/28**

(21) Application number: **81302123.5**

(22) Date of filing: **13.05.81**

(54) **Synthetic crystalline aluminosilicate composition and method for the production thereof.**

(30) Priority: **14.05.80 JP 63653/80**

(43) Date of publication of application:
**18.11.81 Bulletin 81/46**

(45) Publication of the grant of the patent:
**16.10.85 Bulletin 85/42**

(84) Designated Contracting States:
**DE FR GB IT NL**

(56) References cited:
**DE-B-1 264 425**
**DE-B-2 112 223**
**DE-B-2 254 341**
**US-A-3 436 174**
**US-A-3 597 155**
**US-A-3 849 340**

**IND. ENG. CHEM. PROD. RES. DEV., Vol. 17, No. 3, 1978 P.K. BAJAPI et al. "Synthesis of Mordenite Type Zeolite" pages 223 to 227**

(73) Proprietor: **TOA NENRYO KOGYO KABUSHIKI KAISHA**
**1-1 Hitotsubashi, 1-Chome Chiyoda-Ku**
**Tokyo 100 (JP)**

(72) Inventor: **Sakurada, Satoshi**
**745-17, Sashiogi**
**Omiya-shi Saitama-ken (JP)**
Inventor: **Tagaya, Nobuaki**
**2735-4 Ogasa Kasahata**
**Kawagoe shi Saitama-ken (JP)**
Inventor: **Maeshima, Tsugio**
**1428 34, Suneori Tsurugashima machi**
**Iruma gun Saitama-ken (JP)**
Inventor: **Toyoizumi, Takeo**
**1902-5 Kamekubo Ohi-machi**
**Iruma-gun Saitama-ken (JP)**
Inventor: **Numura, Tomio**
**131, Imaizumi**
**Matsuyama-shi Saitama-ken (JP)**
Inventor: **Hasimoto, Takao**
**1902-5 Kamekobo Ohi Machi**
**Iruma-gun Saitama Ken (JP)**

(74) Representative: **Somers, Harold Arnold**
**ESSO Engineering (Europe) Ltd. Patents & Licences Apex Tower High Street**
**New Malden Surrey KT3 4DJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a method for producing a synthetic crystalline aluminosilicate zeolite. More particularly, the present invention relates to a method for the production of mordenite having a high silica content. More particularly, the present invention relates to a method for the production of a crystalline aluminosilicate composition having a high catalytic activity, acid resistance and thermal stability and also having a mordenite framework structure of a high silica/alumina ratio.

Mordenite, which is one of crystalline aluminosilicate zeolites, has an aluminosilicate framework which is based on an infinitely extending three-dimensional network of $SiO_4$ and $AlO_4$ tetrahedra linked to each other by sharing all of the oxygens. In mordenite, the main channels are a 12-membered oxygen ring and the other channels are an 8-membered oxygen ring and the other channels are an 8-membered oxygen ring generally having the following chemical formula:

$$Na_2O \,.\, Al_2O_3 \,.\, 10SiO_2 \,.\, 6H_2O$$

Like other minerals comprising crystalline aluminosilicate, mordenite releases its intercrystalline water without the breaking of the crystalline structure if it is heated to a temperature of about 300—400°C, thereby forming the main channels having dimension of 6Å×7Å (0.6 nm×0.7 nm).

Because of the existence of these channels, therefore, synthetic mordenite is used as catalyst and catalyst carrier for the cracking of various hydrocarbons and also as an effective adsorbent like numerous other crystalline aluminosilicates.

However, when synthetic mordenite is to be used for catalytic dewaxing, production of aromatic compounds and production of hydrocarbons by alcohol conversion, the activity thereof must be further improved by treating the same with a mineral acid to remove aluminium, thereby increasing the silica/alumina ratio. However, the amount of aluminium which can be removed is limited from the viewpoint of thermal stability, acid strength and the maintenance of the crystal structure, since —Si—O—Si— bond in the mordenite crystalline structure is changed to —Si—O—H— by the dealumination reaction. Further, if the dealumination reaction is carried out in such a manner that silica/alumina molar ratio is taken to above a certain level, the crystal structure is destroyed.

Thus, in using mordenite as a catalyst, activity thereof has been limited in the prior art, though a higher activity has been required.

US—A—3597155 discloses contacting with an aqueous mineral acid solution a natural or synthetic mordenite having a chemical composition expressed in terms of mols of oxides as:

$$(1-x)M_{2/n}O:Al_2O_3:ySiO_2:zH_2O$$

wherein M is a metal cation, n is the valence of M, x has a value of from zero to 0.22, y has a value of from about 6 to about 11, and z has a value of from zero to about 7. The resulting three-dimensional crystalline material is not a zeolite in the strictest technical connotation of the term because all true zeolites contain M ions in the ratio of approximately one $M_{2/n}$ per alumina, said M ions being reversibly exchangeable with other materials in ionic form such as ammonium, metals of Groups I and II of the Periodic Table and transition metals, whereas the crystalline materials produced by acid treatment according to US—A—3597155 are not capable of reversibly exchanging the hydrogen or hydronium ions which are believed to reside, after acid treatment, at the sites formerly occupied by M before the acid treatment.

One example of an acid-treated natural mordenite has the composition:

$$0.71M_{2/n}O:Al_2O_3:24.7SiO_2:13.8H_2O$$

US—A—3436174 relates to a method for producing a crystalline aluminosilicate having the mordenite crystal structure. The method comrpises preparing an aqueous reaction mixture having the following composition expressed in molar ratios of oxides:

| | |
|---|---|
| $Na_2O/SiO_2$ | 0.07 to 0.30 |
| $SiO_2/Al_2O_3$ | 10 to 50 |
| $H_2O/Na_2O$ | 6 to 300 |

and reacting the mixture in a closed container under autogeneous pressure at a temperature from 150 to 260°C for a time sufficient to form a zeolite having the mordenite crystal structure (e.g. 16 hours at 170°C). If the temperature is below 200°C, the $H_2O/Na_2O$ ratio is equal to or less than 180.

DE—A—2112223 and the equivalent US—A—3849340 discloses a non-acid-leached synthetic crystalline sodium-zeolite having a $SiO_2/Al_2O_3$ mol ratio of 17.2 and an X-ray powder diffraction pattern similar to the well-known X-ray diffraction pattern of a mordenite. The mole ratios of $Na_2O/Al_2O_3$ and

$Na_2O/SiO_2$ of the sodium-zeolite are not disclosed. It is further disclosed that the $SiO_2/Al_2O_3$ ratio is increased to about 50 by contacting the said sodium zeolite with 12N hydrochloric acid.

After intensive investigations and experiments, a method has been found for producing a crystalline aluminosilicate (from substantially inorganic reaction materials) having an $SiO_2/Al_2O_3$ molar ratio of at least 15 and having a mordenite framework structure which exhibits remarkable shape selectivity and catalyst performance.

A main object of the present invention is to provide a crystalline aluminosilicate composition of mordenite type having a high silica content, i.e. an $SiO_2/Al_2O_3$ molar ratio of at least 15 and also having high thermal stability, acid resistance and catalytic activity.

Another object of the present invention is to provide a process for directly producing a crystalline, aluminosilicate or mordenite type having a high silica content and an $SiO_2/Al_2O_3$ molar ratio of at least 15 in the aqueous reaction mixture in the hydrothermal reaction without necessitating acid treatment.

According to the present invention, the above objects can be attained effectively.

The present invention provides a method for producing a synthetic crystalline aluminosilicate zeolite composition which comprises preparing an aqueous reaction mixture substantially comprising inorganic reaction materials of the following molar ratios of oxides:

| | |
|---|---|
| $SiO_2/Al_2O_3$ | 16—50 |
| $Na_2O/SiO_2$ | 0.05—1.5 |
| $(Na_2O+M_{2/n}O)/SiO_2$ | 0.05—1.5 |
| $H_2O/(Na_2O+M_{2/n}O)$ | 300—1200 |

wherein M is at least one metallic cation and *n* is the valence of the metallic cation, maintaining the mixture at a temperature in the range of from 150 to 200°C under an autogeneous pressure for from 10 to 40 hours, and recovering from the mixture a crystalline aluminosilicate zeolite composition having the following chemical composition (in terms of molar ratios of oxides):

$$0.5 \text{ to } 3.0\ M_{2/n}O:Al_2O_3:15 \text{ to } 30\ SiO_2:0 \text{ to } 50\ H_2O$$

and an X-ray powder diffraction pattern as follows:

| Interplanar spacing, d Å or m×$10^{-10}$ | Relative intensity |
|---|---|
| 13.9±0.2 | Strong |
| 9.15±0.15 | Very strong |
| 6.55±0.07 | Strong |
| 4.51±0.03 | Strong |
| 3.97±0.03 | Very strong |
| 3.46±0.02 | Strong |
| 3.37±0.02 | Strong |
| 3.22±0.05 | Strong |

The above values of interplanar spacing and relative intensities were determined by standard techniques. The radiation was the K-alpha doublet of copper, and a scintillation counter spectrometer with a strip chart pen recorder was used. The peak heights I and the positions as a function of 2 times theta, where theta is the Bragg angle, were read from the spectrometer chart. The relative intensities, $I/I_o$, where $I_o$ is the intensity of the strongest line or peak, and d, the interplanar spacing in Angstrom units, corresponding to the recorded lines, were calculated. The crystalline aluminosilicate of the present invention has the highest relative intensity at an interplanar spacing of 9.15Å (0.915 nm). From the X-ray powder diffraction pattern, it is understood that the framework structure of the crystalline aluminosilicate of the present invention is the same as a framework structure of conventional mordenite.

Figures 1 to 5 show X-ray diffraction patterns of the synthetic, crystalline aluminosilicate compositions obtained by the method of the present invention.

Figure 6 shows carbon number distribution of a liquid hydrocarbon product according to gas chromatography.

A preferred composition of the aqueous reaction mixture is as follows (molar ratios of oxides):

| | |
|---|---|
| $SiO_2/Al_2O_3$ | 16—35 |
| $Na_2O/SiO_2$ | 0.05—0.50 |
| $(Na_2O+M_{2/n}O)/SiO_2$ | 0.05—0.50 |
| $H_2O/(Na_2O+M_{2/n}O)$ | 300—1000 |

The most suitable range of the proportion of the components of the aqueous mixture is as follows (molar ratios of oxides):

| | |
|---|---|
| $SiO_2/Al_2O_3$ | 16—30 |
| $Na_2O/SiO_2$ | 0.08—0.25 |
| $(Na_2O+M_{2/n}O)/SiO_2$ | 0.08—0.25 |
| $H_2O/(Na_2O+M_{2/n}O)$ | 300—1000 |

wherein M is at least one metallic cation selected from the group consisting of Groups I and II in the Periodic Table, particularly, sodium, lithium, barium, calcium and strontium, most preferably lithium and *n* represents the valence of the metallic cation. $M_{2/n}O$ and $Na_2O$ are both in the free form. They are generally in the from of hydroxide or an extremely weak acid salt effective in the zeolite synthesis such as aluminate or silicate. "Free $Na_2O$" can be controlled by the addition of aluminum sulfate, sulfuric acid or nitric acid.

The oxides of the above composition used in the preparation of the aqueous reaction mixture are those generally used in the preparation of synthetic zeolites. For example, silica sources are sodium silicate, silica gel, silicic acid, dissolved silica, aqueous colloidal silica sol, powdery silica and amorphous silica. As alumina sources, there may be used various aluminum salts such as activated alumina, γ-alumina, alumina trihydrate, sodium aluminate and aluminum chloride, nitrate and sulfate. The metal oxide of the formula $M_{2/n}O$ is added to the reaction mixture in the form of a water-soluble salt or hydroxide thereof. $Na_2O$ used as sodium cation source is added in the form of sodium hydroxide, sodium aluminate or sodium silicate. $Li_2O$ used as lithium cation source is added in the form of its hydroxide, halide, sulfate, nitrate, chlorate ($LiClO_4$) or the like. The aqueous reaction mixture is prepared by mixing the above silica source, alumina source, alkali source and water together. Particularly preferred silica sources are sodium silicate, water glass and colloidal silica. Particularly preferred alumina sources are sodium aluminate and aluminum sulfate.

In the production of the crystalline aluminosilicate having a high silica content of the present invention, water content of the aqueous reaction mixture is important. As described above, the reaction mixture should contain at least 300 moles, preferably at least 300—500 moles, of water per mole of $Na_2O+M_{2/n}O$. By controlling the water content in the above-described range, the mixing and stirring of the gel reactants are facilitated.

As described above, after mixing the reagents, the reaction mixture is maintained at a temperature in the range of about 150—200°C for about 10—40 hours under an autogenous pressure.

The crystallinity of the crystallized product can be further improved and the formation of amorphous aluminosilicate can be prevented by adding a mineralizer to the aqueous reaction mixture in the crystallization. As the mineralizers, there may be used salts of alkali metals and alkaline earth metals such as NaCl, $Na_2CO_3$, $Na_2SO_4$, $Na_2SeO_4$, KCl, KBr, KF, $BaCl_2$ and $BaBr_2$. The amount of the mineralizer is preferably such that molar ratio of $X^-/SiO_2$ wherein $X^-$ represents an anion of the salt used as the mineralizer (if the anion has a valence of *n*, one valence equivalent thereof is taken), is in the range of about 0.01—20.

The resulting crystalline aluminosilicate is separated out of the solution by filtration, washed with water and dried. After drying, the product is calcined at a temperature of above 200°C in air or an inert gas atmosphere to remove intercrystalline water. Thus, the dehydrated product is useful as a catalyst for chemical reaction or catalyst carrier. Further, it is preferred to remove or to exchange at least a part of the cation in the product by thermal treatment and/or ion exchange. The cation-exchanged product is effective particularly as a catalyst for hydrocarbon conversion. A cation species used for the ion-exchange may be selected depending upon the intended reaction. Among them, at least one cation selected from the group consisting of metals of Groups IIa, IIIa, IVa, Ib, IIb, IIIb, IVb and VIII is preferred. In addition, the cation in the product can be exchanged with hydrogen ion by the treatment with an acid or by the combination of the ion exchange with $NH_4^+$ with the thermal treatment. Exchanging cations preferred for the cracking,

isomerization and alkylation of hydrocarbons are hydrogen ion and cations of metals of Group VIII in the Periodic Table.

The cation exchange may be effected by contacting the crystallized product with salt(s) of desired exchanging cation(s). In this step, various salts may be used. Particularly, chlorides, nitrates, sulfates and acetates are preferred.

Framework aluminum can be removed and silica/alumina ratio can be increased to above 30 by contacting the hydrogen ion-exchanged crystalline aluminosilicate prepared by the method of the invention with an acid extraction. As a result, the acid strength and catalytic activity can be improved further. In the case of mordenite zeolite used in the prior art, it is difficult to attain a high silica/alumina ratio by a conventional extraction treatment with an acid. Under the circumstances as above, specific processes such as a process wherein the combination of an acid extraction with steaming is employed or a process wherein multi-stage extraction treatment with an acid have been proposed.

However, when the crystalline aluminosilicate or mordenite type having a high silica content prepared according to the method of the present invention is used, the silica/alumina ratio can be increased remarkably without destroying the crystalline structure as will be described below.

As preferred acids used in the extraction treatment, there may be mentioned various mineral acids such as hydrochloric acid, sulfuric acid, phosphoric acid and nitric acid as well as organic acids such as acetic acid and formic acid. Among them, hydrochloric acid is particularly preferred. The concentration of the acid used is in the range of 1 to 12 N. The temperature in the process of the extraction with an acid ranges from room temperature to about 95°C. The higher the temperature, the better. For example, by effecting the acid treatment with 3N hydrochloric acid at 90°C for 4 hours, silica/alumina molar ratio of the starting crystalline aluminosilicate (mordenite type) can be converted from 20 to 35. It has been found that after this treatment, crystallinity of the product was substantially unchanged as compared with that of the starting material. By the above-described process, crystalline aluminosilicate having a silica/alumina molar ratio of at least about 600—700 can be obtained.

Thus, the present invention relates to a process for the production of a synthetic crystalline aluminosilicate composition having the following chemical composition (in terms of molar ratios of oxides):

$$0.5\text{—}3M_{2/n}O \cdot Al_2O_3 \cdot 15\text{—}30SiO_2 \cdot 0\text{—}50H_2O$$

wherein M represents a metallic cation and *n* is the valence of the metallic cation and also having an X-ray powder diffraction pattern having d-spacing as shown in Table 1, and a process for the production of said composition. According to the present invention, crystalline aluminosilicate (mordenite type) having a high silica content and a high commercial value can be provided.

The following examples further illustrate the crystalline aluminosilicate of the present invention.

Example 1

9.1 g of aluminum sulfate, 1.1 g of concentrated sulfuric acid and 14 g of sodium chloride were dissolved in 150 g of pure water. 49 g of water-glass (No. 3 water-glass: Japanese Industrial Standard) was added to the solution to obtain an aqueous reaction mixture having a composition of

$$1.5Na_2O \cdot Al_2O_3 \cdot 17.0SiO_2 \cdot 641H_2O.$$

The mixture was aged at room temperature for about one hour and then charged in an autoclave. The temperature was elevated rapidly and the mixture was maintained to 180°C for 20 hours. The resulting product was cooled at room temperature, filtered, washed thoroughly with water and dried at 110°C.

A part of the solid product was calcined at 700°C in air. After allowing the same to adsorb water, it was subjected to chemical analysis to obtain the following results:

| Chemical composition | Wt. % |
|---|---|
| $SiO_2$ | 74.4 |
| $Al_2O_3$ | 7.26 |
| $Na_2O$ | 5.04 |
| Ignition loss (600°C, 3 hrs.) | 11.0 |

The chemical composition was as follows (in terms of molar ratios of oxides):

$$1.14Na_2O \cdot Al_2O_3 \cdot 17.5SiO_2 \cdot 8.6H_2O$$

A part of the product was subjected to X-ray analysis to obtain the results shown in Table 2 and Fig. 1. From the results, it was proved that the product having a crystal structure of mordenite was a new synthetic, crystalline aluminosilicate (HSM).

TABLE 2

| Interplanar spacing d (Å or $m\times10^{-10}$) | Relative intensity |
|---|---|
| 13.59±0.2 | S. |
| 10.16±0.2 | S. |
| 9.15±0.1 | V.S. |
| 6.55±0.05 | S. |
| 6.36±0.05 | S. |
| 6.03±0.05 | S. |
| 5.80±0.05 | S. |
| 5.08±0.05 | S. |
| 4.51±0.02 | S. |
| 3.97±0.02 | V.S. |
| 3.81±0.02 | S. |
| 3.75±0.02 | S. |
| 3.46±0.01 | S. |
| 3.37±0.01 | S. |
| 3.22±0.01 | S. |
| 2.89±0.01 | S. |
| 2.51±0.01 | S. |
| 2.03±0.01 | S. |
| 2.01±0.01 | S. |
| 1.87±0.01 | S. |

S.=Strong, V.S.=Very strong.

Example 2

6.7 g of aluminum sulfate, 1.97 g of concentrated sulfuric acid and 14 g of sodium chloride were dissolved in 150 g of pure water. 49 g of water-glass (No. 3 water-glass) was added to the solution to obtain an aqueous reaction mixture having a composition of

$$2.53Na_2O:Al_2O_3:23.1SiO_2:990H_2O.$$

The mixture was aged at room temperature for about one hour and then charged in an autoclave. The temperature was elevated rapidly and the mixture was maintained at 180°C for 20 hours. The resulting product was cooled to room temperature, filtered, washed thoroughly with water and dried at 110°C.

A part of the solid product was calcined at 700°C in air. After allowing the same to adsorb water, it was subjected to chemical analysis to obtain the following results.

| Chemical composition | Wt. % |
|---|---|
| $SiO_2$ | 79.1 |
| $Al_2O_3$ | 5.96 |
| $Na_2O$ | 3.51 |
| Ignition loss (600°C, 3 hrs.) | 10.0 |

The chemical composition was as follows (in terms of molar ratios of oxides).

$$0.98Na_2O \cdot Al_2O_3 \cdot 22.6SiO_2 \cdot 9.5H_2O$$

A part of the product was subjected to X-ray analysis to obtain the results shown in Table 2 and Fig. 2. From the results, it was proved that the product had a crystal structure of mordenite.

Example 3

26.2 g of aluminum sulfate, 10.4 g of concentrated sulfuric acid and 60 g of sodium chloride were dissolved in 542 g of pure water. 207 g of water-glass (No. 3 water-glass) was added to the solution to obtain an aqueous reaction mixture having a composition of

$$2.54Na_2O \cdot Al_2O_3 \cdot 25.3SiO_2 \cdot 955H_2O.$$

The mixture was aged at room temperature for about one hour and then charged in an autoclave. The temperature was elevated rapidly and the mixture was maintained at 180°C for 20 hours. The resulting product was cooled to room temperature, filtered, washed thoroughly with water and dried at 110°C for about 16 hours. A part of the solid product was subjected to the X-ray analysis to obtain the results shown in Table 2 and Fig. 3.

From the results, it was proved that the product had a crystal structure of mordenite. A part of the product was calcined at 700°C in air, then allowed to adsorb water and subjected to chemical analysis to obtain the following results.

| Chemical composition | Wt. % |
|---|---|
| $SiO_2$ | 82.3 |
| $Al_2O_3$ | 5.43 |
| $Na_2O$ | 3.31 |
| Ignition loss (660°C, 3 hrs.) | 8.9 |

The chemical composition was as follows (in terms of molar ratios of oxides).

$$1.00Na_2OAl_2O_3 \cdot 25.8SiO_2 \cdot 9.3H_2O.$$

Example 4

48.2 g of aluminum sulfate, 4.0 g of concentrated sulfuric acid and 70 g of sodium chloride were dissolved in 750 g of pure water. 245 g of water-glass (No. 3 water-glass) was added to the solution to obtain a mixture having a composition of

$$1.5Na_2O \cdot Al_2O_3 \cdot 16.0SiO_2 \cdot 535H_2O.$$

The mixture was aged at room temperature for about one hour and then charged in an autoclave. The temperature was elevated rapidly and the mixture was kept at 180°C for 20 hours. The resulting product was cooled to room temperature, filtered, washed thoroughly with water and dried at 110°C. After the drying, a part of the solid product was calcined at 700°C in air, allowed to adsorb water and subjected to chemical analysis to obtain the following results.

| Chemical composition | Wt. % |
|---|---|
| $SiO_2$ | 8.54 |
| $Al_2O_3$ | 75.4 |
| $Na_2O$ | 5.79 |
| Ignition loss (600°C, 3 hrs.) | 10.3 |

From the above analytical results, the synthetic, crystalline aluminosilicate in this example can be represented in terms of molar ratios of oxides as follows:

$$1.11Na_2O \cdot Al_2O_3 \cdot 150SiO_2 \cdot 6.8H_2O$$

From the results of X-ray diffraction (Fig. 4), it was proved that the product had a crystal structure of mordenite type.

Example 5

77 g of aluminum sulfate, 14.7 g of concentrated sulfuric acid and 140 g of sodium chloride were dissolved in 1500 g of pure water. 490 g of water-glass (No. 3 water-glass) was added to the solution to obtain an aqueous reaction mixture having a composition of

$$2.2Na_2O \cdot Al_2O_3 \cdot 20SiO_2 \cdot 718H_2O.$$

The mixture was aged at room temperature for about one hour and then charged in an autoclave. The temperature was elevated rapidly and the mixture was maintained at 180°C for 20 hours. The resulting product was cooled to room temperature, filtered, washed thoroughly with water and dried at 110°C.

After the drying, a part of the solid product was calcined at 700°C in air, allowed to adsorb water and subjected to chemical analysis to obtain the following results:

| Chemical composition | Wt. % |
|---|---|
| $SiO_2$ | 80.7 |
| $Al_2O_3$ | 6.64 |
| $Na_2O$ | 4.52 |
| Ignition loss (600°C, 3 hrs.) | 8.2 |

From the above analytical results, the synthetic, crystalline aluminosilicate in this example can be represented in terms of molar ratios of oxides as follows:

$$1.12Na_2O \cdot Al_2O_3 \cdot 20.6SiO_2 \cdot 7H_2O$$

From the results of X-ray diffraction (Fig. 5), it was proved that the solid product had a crystalline structure of mordenite type.

Example 6

9.1 g of aluminum sulfate, 1.1 g of concentrated sulfuric acid and 14 g of sodium chloride were dissolved in 150 g of pure water, 49 g of water-glass (No. 3 water-glass) and 7.6 g of $LiOH \cdot H_2O$ were added to the solution. The mixture had a composition of

$$0.5Li_2O \cdot 0.98Na_2O \cdot Al_2O_3 \cdot 17.0SiO_2 \cdot 641H_2O.$$

The mixture was aged at room temperature for about one hour and then charged in an autoclave. The mixture was maintained at 180°C for 20 hours. The resulting product was cooled to room temperature, filtered, washed thoroughly with water and dried at 110°C for about 16 hours. A part of the product was calcined at 700°C in air, allowed to adsorb water and subjected to chemical analysis to obtain the following results:

| Chemical composition | Wt. % |
|---|---|
| $SiO_2$ | 75.1 |
| $Al_2O_3$ | 7.47 |
| $Na_2O$ | 3.86 |
| $Li_2O$ | 0.40 |
| Ignition loss (600°C, 3 hrs.) | 11.1 |

From the above analytical results, the following composition in terms of molar ratios of oxides was obtained:

$$0.18Li_2O \cdot 0.85Na_2O \cdot Al_2O_3 \cdot 17.1SiO_2 \cdot 8.4H_2O$$

A part of the product was subjected to the X-ray analysis to obtain the same results as in Example 1. From the results, it was proved that the product had a crystalline structure of mordenite.

Example 7

9.1 g of aluminum sulfate, 1.1 g of concentrated sulfuric acid and 14 g of sodium chloride were dissolved in 150 g of pure water. 49 g of water-glass, (No. 3 water-glass) 20 g of $CaCl_2$ and 14 g of NaOH were added to the solution to obtain an aqueous reaction mixture having a composition of

$$0.51CaO \cdot 0.98Na_2O \cdot Al_2O_3 \cdot 17.0SiO_2 \cdot 641H_2O.$$

The mixture was aged at room temperature for about one hour, charged in an autoclave and maintained at 180°C for 20 hours.

The resulting product was cooled to room temperature, filtered, washed thoroughly with water and dried at 110°C for about 16 hours. A part of the solid product was calcined at 700°C in air, allowed to adsorb water and subjected to the chemical analysis to obtain the following results:

| Chemical composition | Wt. % |
|---|---|
| $SiO_2$ | 75.8 |
| $Al_2O_3$ | 7.81 |
| $Na_2O$ | 4.51 |
| CaO | 7.81 |
| Ignition loss (600°C, 3 hrs.) | 10.75 |

From the above analytical results, the following composition in terms of molar ratios of oxides was obtained:

$$0.17CaO \cdot 0.95Na_2O \cdot Al_2O_3 \cdot 16.5SiO_2 \cdot 7.8H_2O$$

Example 8

Thermal stability of the new crystalline aluminosilicate (HSM) obtained in Example 1 was compared with that of a commercially available, synthetic mordenite.

An X-ray sample-heating device capable of scanning a specific angle of diffraction while heating is effected at a rate of 5°C/min in air was used. The operation was effected by standard technique of X-ray powder diffraction.

Radiation was the K-a doublet of copper and the intensity of diffraction line at 25.7°2θ was measured.

The measurement was continued while the temperature was elevated, taking an intensity of diffraction line at 2θ=25.7° at 300°C as 100. Thus, a point at which the relative intensity of the line is lowered to below 100 was defined as thermal resisting temperature and a point at which the relative value is lowered to 50 was defined as limiting thermal resisting temperature. The results of the measurement are shown in Table 3.

TABLE 3

| | Thermal resistance temp. (°C) | Limiting thermal resistance temp. (°C) |
|---|---|---|
| Commercially available Zeolon 100H | 740 | 880 |
| Mordenite of a high silica content (HSM) | 810 | 970 |

It is apparent from the above results that mordenite having a high silica content after the synthesis has an improved thermal stability due to the large number of bands —Si—O—Si—.

Example 9

Activity of the new crystalline aluminosilicate obtained in Example 1 (HSM) was compared with that of a commercially available synthetic mordenite. The cracking reaction of n-hexane was effected in a glass reactor.

The new crystalline aluminosilicate (HSM) was ion-exchanged with 5% $NH_4Cl$ solution at 80°C for 1.5 hours. The ion-exchange was repeated four times. Then, the $NH_4$-type HSM was calcined at 600°C for 3 hours to obtain H-type zeolite.

(Preparation of alumina binder)

A solution of 202 g of sodium aluminate ($NaAlO_2$) in 264 g of water ($H_2O$) was added to an ion-exchanged water (4800 g) kept at 70°C. Then, a solution of 288 g of aluminum sulfate [$Al_2(SO_4)_3 \cdot 17.8H_2O$] in 507 g of water ($H_2O$) was added thereto and the whole was aged for one hour. Thereafter, the mixture was filtered and washed to obtain an aluminum hydrogel.

(Preparation of pellets)

The new crystalline aluminosilicate (HSM) of H-type obtained as above was mixed with the alumina binder in such a ratio that weight ratio of them after the calcination would be 7:3. The mixture was kneaded well together with water and then extrusion-molded to obtain pellets of a diameter of 1.5 mm. The pellets were dried and then calcined at 600°C for 3 hours to obtain a catalyst.

(Evaluation of activity)

n-Hexane cracking reaction was carried out as follows:

3.2 ml of the catalyst ground into 16/30 mesh* was charged in a glass reactor. Then, 3.2 ml of silica chips also ground into 16/30 mesh* was placed over the catalyst bed to facilitate the diffusion and heating of the reactants. The catalyst bed was kept at 300°C.

n-Hexane was charged in a saturator kept at 10°C. Nitrogen gas was introduced therein as a carrier gas so that n-hexane concentration in the nitrogen gas would be 10%.

After a certain period of time has passed after the introduction of the n-hexane/nitrogen mixture in the reactor, a residual amount of n-hexane was measured by gas chromatograph. From the residual amount and the amount of n-hexane in the feed, conversion was calculated.

Thereafter, air was introduced in the reactor, and carbonaceous substances deposited on the catalyst were burnt at 500°C to recover the fresh condition of the catalyst. Space velocity was altered and the experiment was effected again.

Conversions of n-hexane at 300°C and 275°C were thus determined to obtain the results shown in Table 4. From the results, it is understood that the new crystalline aluminosilicate obtained in Example 1 had a high n-hexane cracking activity.

---

*16/30 mesh is 1.19 to 0.595 mm.

TABLE 4
n-Hexane-cracking activities

| | $k(\ln\frac{1}{1-x})$ | | |
|---|---|---|---|
| Reaction temp. (°C) | 300 | 300 | 275 |
| Contact time $(HR^{-1})/1/S.V\times10^2$ | 1.67 | 0.83 | 1.67 |
| Time after initiation of the reaction (min.) | | | |
| 5 | −(*1) | 3.22 | −(*1) |
| 10 | −(*1) | 0.94 | 1.51 |
| 20 | 0.94 | 0.36 | 0.40 |
| 30 | 0.41 | | 0.33 |
| 45 | 0.26 | | |
| 60 | 0.16 | | |

(*1) 100% cracking of n-hexane
S.V. in explanation of contact time means volume hourly space velocity.

Example 10

Activity of the new crystalline aluminosilicate (HSM) obtained in Example 1 was compared with that of a commercially available synthetic mordenite. Alcohol conversion reaction was effected in a glass reactor.

3.0 ml of the aluminosilicate zeolite of 16/30 mesh* obtained in Example 9 was mixed thoroughly with 3.0 ml of silica chips also ground into 16/30 mesh*. The mixture was charged in a glass reactor.

The catalyst bed was kept in nitrogen stream at 500°C for 2 hours. Then, the temperature was lowered to 370°C in the nitrogen stream and the whole was kept at that temperature. Then, the supply of nitrogen stream was stopped and methanol was introduced rapidly at a rate of 4.5 ml/hr in the reactor.

After one hour, the methanol injection was stopped and nitrogen was introduced again therein to purge the reaction product and unreacted reactants from the reactor. The product discharge from the reactor was cooled with water at 20°C to separate the same into a gaseous product and a liquid product (mixture of water and hydrocarbons).

The resulting liquid product had the following composition:

| | |
|---|---|
| Hydrocarbons | 0.5 g |
| Water | 1.93 g |

The theoretical quantity of water to be formed was 1.99 g. Yield of water was thus 97%.

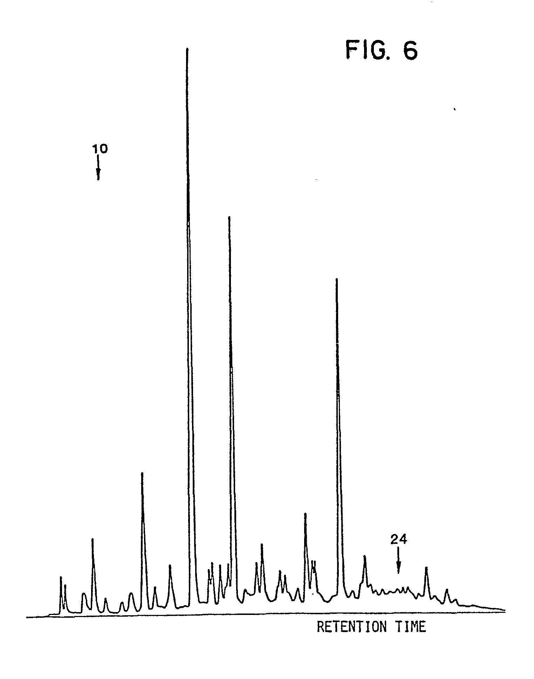

Carbon number distribution of the liquid hydrocarbons according to gas chromatography is shown in Fig. 6.

The composition of the gaseous product was as shown in Table 5.

---

*16/30 mesh is 1.19 to 0.595 mm.

TABLE 5
Gas composition

| | |
|---|---|
| $C_1$ | 24.5% |
| $C_2$ | 23.0% |
| $C_3$ | 19.3% |
| $C_3$= | 10.5% |
| $iC_4$ | 6.3% |
| $nC_4$ | 4.6% |
| $C_4$= | 7.1% |
| $iC_5$ | 2.3% |
| $nC_5$ | 0.4% |
| $C_5$= | 1.9% |

Comparative Example

The commercially available, synthetic mordenite (molded product of Zeolon 100H) used in Example 8 was ground into 16/30 mesh* and subjected to the reaction in the same manner as in Example 9.

The liquid product comprised substantially methanol and contained only a trace of hydrocarbons.

From the above results, it is apparent that the synthetic, crystalline aluminosilicate (HSM) prepared by the process of the present invention has a high catalytic activity.

The Periodic Table of the elements for the purpose of this patent specification is the Periodic Table published by the Fisher Scientific Company, Catalogue No. 5-702-10.

Mesh sizes referred to herein are those of the American Society for Testing Materials (ASTM), Standard E-11-70.

**Claims**

1. A method for producing a synthetic crystalline aluminosilicate zeolite composition which comprises preparing an aqueous reaction mixture substantially comprising inorganic reaction materials of the following molar ratios of oxides:

| | |
|---|---|
| $SiO_2/Al_2O_3$ | 16—50 |
| $Na_2O/SiO_2$ | 0.05—1.5 |
| $(Na_2O+M_{2/n}O)/SiO_2$ | 0.05—1.5 |
| $H_2O/(Na_2O+M_{2/n}O)$ | 300—1200 |

wherein M is at least one metallic cation and *n* is the valence of the metallic cation, maintaining the mixture at a temperature in the range of from 150 to 200°C under an autogenous pressure for from 10 to 40 hours, and recovering from the mixture a crystalline aluminosilicate zeolite composition having the following chemical composition (in terms of molar ratios of oxides):

$$0.5 \text{ to } 3.0\ M_{2/n}O:Al_2O_3:15 \text{ to } 30\ SiO_2:0 \text{ to } 50\ H_2O$$

and an X-ray powder diffraction pattern as follows:

*16/30 mesh is 1.19 to 0.595 mm.

| Interplanar spacing, d Å or m×$10^{-10}$ | Relative intensity |
|---|---|
| 13.9±0.2 | Strong |
| 9.15±0.15 | Very strong |
| 6.55±0.07 | Strong |
| 4.51±0.03 | Strong |
| 3.97±0.03 | Very strong |
| 3.46±0.02 | Strong |
| 3.37±0.02 | Strong |
| 3.22±0.05 | Strong |

2. A method according to claim 1 wherein the aqueous reaction mixture has the following composition (in terms of molar ratios of oxides):

| | |
|---|---|
| $SiO_2/Al_2O_3$ | 16—30 |
| $Na_2O/SiO_2$ | 0.08—0.25 |
| $(Na_2O+M_{2/n}O)/SiO_2$ | 0.08—0.25 |
| $H_2O/(Na_2O+M_{2/n}O)$ | 300—1000 |

3. A method according to claim 1 or claim 2 wherein M in the aqueous reaction mixture is at least one member selected from alkali metal cations and alkaline earth metal cations.

4. A method according to claim 3 wherein M is the aqueous reaction mixture is a mixture comprising sodium cation and lithium cation.

5. A method according to any one of claims 1, 2, 3 or 4 wherein the composition of the aqueous reaction mixture, expressed in terms of molar ratios of oxides, is one of the following:

(a) $1.5Na_2O:Al_2O_3:17.0SiO_2:641H_2O$; or
(b) $2.53Na_2O:Al_2O_3:23.1SiO_2:990H_2O$; or
(c) $2.54Na_2O:Al_2O_3:25.3SiO_2:955H_2O$

and crystallization is effected at about 180°C for about 20 hours.

6. A method for producing a hydrogen ion-exchanged crystalline aluminosilicate zeolite composition which comprises preparing a synthetic, crystalline aluminosilicate composition by the method of any one of claims 1 to 5 and contacting the recovered aluminosilicate composition with an acid.

7. A method of producing a synthetic crystalline aluminosilicate composition of relatively high silica to alumina ratio and having an X-ray powder diffraction pattern having a *d*-spacing as in claim 1 comprising preparing a hydrogen ion-exchanged crystalline aluminosilicate composition by the method of claim 6, contacting this composition with an acid, and recovering a hydrogen ion-exchanged crystalline aluminosilicate composition of increased silica to alumina ratio with an X-ray powder diffraction pattern having a *d*-spacing as in claim 1.

**Patentansprüche**

1. Verfahren zur Herstellung einer synthetischen kristallinen Aluminiumsilikatzeolith-Zusammensetzung, dadurch gekennzeichnet, daß man eine wässrige Reaktionsmichung aus im wesentliche anorganischen Reaktionsmaterialien in den folgenden molaren Verhältnissen der Oxide herstellt:

| | |
|---|---|
| $SiO_2/Al_2O_3$ | 16—50 |
| $Na_2O/SiO_2$ | 0,05—1,5 |
| $(Na_2O+M_{2/n}O)/SiO_2$ | 0,05—1,5 |
| $H_2O/(Na_2O+M_{2/n}O)$ | 300—1200, |

wobei M mindestens ein metallisches Kation ist und n die Wertigkeit des metallischen Kations ist, die Mischung über 10 bis 40 Stunden unter autogenem Druck auf einer Temperatur im Bereich von 150 bis 200°C hält und aus der Mischung eine kristalline Aluminiumsilikatzeolith-Zusammensetzung mit der folgenden chemischen Zusammensetzung (ausgedrückt als Molverhältnisse der Oxide):

$$0{,}5 \text{ bis } 3{,}0\ M_{2/n}O:Al_2O_3:15 \text{ bis } 30\ SiO_2:0 \text{ bis } 50\ H_2O$$

und folgendem Pulverröntgenbeugungsspektrum gewinnt:

| Netzebenenabstand, d Å oder $m\times10^{-10}$ | Relative Intensität |
|---|---|
| 13,9±0,2 | stark |
| 9,15±0,15 | sehr stark |
| 6,55±0,07 | stark |
| 4,51±0,03 | stark |
| 3,97±0,03 | sehr stark |
| 3,46±0,02 | stark |
| 3,37±0,02 | stark |
| 3,22±0,05 | stark |

2. Verfahren nach Anspruch 1, dadurch gekennzeichnet, daß die wässrige Reaktionsmischung die folgende Zusammensetzung (ausgedrückt als molare Verhältnisse der Oxide) besitzt:

| | |
|---|---|
| $SiO_2/Al_2O_3$ | 16—30 |
| $Na_2O/SiO_2$ | 0,08—0,25 |
| $(Na_2O+M_{2/n}O)/SiO_2$ | 0,08—0,25 |
| $H_2O/(Na_2O+M_{2/n}O)$ | 300—1000. |

3. Verfahren nach Anspruch 1 oder 2, dadurch gekennzeichnet, daß M in der wässrigen Reaktionsmischung mindestens ein Mitglied ausgewählt aus Alkalimetallkationen und Erdalkalimetallkationen ist.

4. Verfahren nach Anspruch 3, dadurch gekennzeichnet, daß M in der wässrigen Reaktionsmischung eine Mischung ist, die Natriumkationen und Lithiumkationen umfaßt.

5. Verfahren nach einem der Ansprüche 1, 2, 3 oder 4, dadurch gekennzeichnet, daß die Zusammensetzung der wässrigen Reaktionsmischung, ausgedrückt in Form der molaren Verhältnisse der Oxide, eine der folgenden ist:

(a) $1{,}5Na_2O:Al_2O_3:17{,}0SiO_2:641H_2O$ oder
(b) $2{,}53Na_2O:Al_2O_3:23{,}1SiO_2:990H_2O$ oder
(c) $2{,}54Na_2O:Al_2O_3:25{,}3SiO_2:955H_2O$

und die Kristallisation für etwa 20 Stunden bei etwa 180°C bewirkt wird.

6. Verfahren zur Herstellung einer wasserstoffionenausgetauschten kristallinen Aluminiumsilikatzeolith-Zusammensetzung, das dadurch gekennzeichnet ist, daß man eine synthetische, kristalline Aluminiumsilikatzusammensetzung gemäß dem Verfahren nach einem der Ansprüche 1 bis 5 herstellt und die gewonnene Aluminiumsilikatzusammensetzung mit einer Säure kontaktiert.

7. Verfahren zur Herstellung einer synthetischen kristallinen Aluminiumsilikatzusammensetzung mit verhältnismäßig hohem Siliciumdioxid-Aluminiumoxid-Verhältnis und einem Pulverröntgenbeugungsspektrum mit d-Abständen gemäß Anspruch 1, dadurch gekennzeichnet, daß man eine wasserstoffionenausgetauschte kristalline Aluminiumsilikatzusammensetzung nach dem Verfahren gemäß Anspruch 6 herstellt, diese Zusammensetzung mit einer Säure kontaktiert und eine wasserstoffionenausgetauschte kristalline Aluminiumsilikatzusammensetzung mit erhöhtem Siliciumdioxid-Aluminiumoxid-Verhältnis mit einem Pulverröntgenbeugungsspektrum mit d-Abständen wie in Anspruch 1 gewinnt.

**Revendications**

1. Procédé de production d'une composition d'aluminosilicate cristalline synthétique du type zéolite qui comprend la préparation d'un mélange réactionnel aqueux comprenant essentiellement des substances réactives minérales dans les rapports molaries d'oxydes suivants:

| | |
|---|---|
| $SiO_2/Al_2O_3$ | 16—50 |
| $Na_2O/SiO_2$ | 0,05—1,5 |
| $(Na_2O+M_{2/n}O)/SiO_2$ | 0,05—1,5 |
| $H_2O/(Na_2O+M_{2/n}O)$ | 300—1200 |

où M est au moins un cation métallique et *n* est la valence du cation métallique, le maintien du mélange à une température comprise tnre 150 et 200°C sous la pression spontanée pendant 10 à 409 heures et la séparation, à partir du mélange, d'un aluminosilicate cristallin du type zéolite ayant la composition chimique suivante (exprimée en rapports molaires d'oxydes):

$$0{,}5 \text{ à } 3{,}0\ M_{2/n}O:Al_2O_3:15 \text{ à } 30\ SiO_2:0 \text{ à } 50\ H_2O$$

et un diagramme de diffraction de rayons X de poudre comme suit:

| Distance des plans réticulaires, d Å ou $m\times10^{-10}$ | Intensité relative |
|---|---|
| 13,9±0,2 | forte |
| 9,15±0,15 | très forte |
| 6,55±0,07 | forte |
| 4,51±0,03 | forte |
| 3,97±0,03 | très forte |
| 3,46±0,02 | forte |
| 3,37±0,02 | forte |
| 3,22±0,05 | forte |

2. Procédé selon la revendication 1, dans lequel le mélange réactionnel aqueux a la composition suivante (exprimé en rapports molaries d'oxydes):

| | |
|---|---|
| $SiO_2/Al_2O_3$ | 16—30 |
| $Na_2O/SiO_2$ | 0,08—0,25 |
| $(Na_2O+M_{2/n}O)/SiO_2$ | 0,08—0,25 |
| $H_2O/(Na_2O+M_{2/n}O)$ | 300—1000 |

3. Procédé selon la revendication 1 ou 2, dans lequel M dans le mélange réactionnel aqueux est au moins un cation choisi parmi les cations de métaux alcalins et les cations de métaux alcalino-terreux.

4. Procédé selon la revendication 3, dans lequel M dans le mélange réactionnel aqueux est un mélange de cations sodium et de cations lithium.

5. Procédé selon l'une quelconque des revendications 1 à 4, dans lequel la composition du mélange réactionnel aqueux, exprimée en termes de rapports molaires d'oxydes, est une des suivantes:

(a) $1{,}5Na_2O:Al_2O_3:17{,}0SiO_2:641H_2O$; ou
(b) $2{,}53Na_2O:Al_2O_3:23{,}1SiO_2:990H_2O$; ou
(c) $2{,}54Na_2O:Al_2O_3:25{,}3SiO_2:955H_2O$

et la cristallisation est réalisée à environ 180°C pendant environ 20 heures.

6. Procédé de production d'une composition d'aluminosilicate cristallin du type zéolite ayant subi un échange avec l'ion hydrogène qui comprend la préparation d'une composition d'aluminosilicate cristallin synthétique par le procédé selon l'une quelconque des revendications 1 à 5 et la mise en contact de la composition d'aluminosilicate obtenue avec un acide.

7. Procédé de production d'une composition d'aluminosilicate cristalline synthétique ayant un rapport silice à alumine relativement élevé et ayant un diagramme de diffraction de rayons X de poudre correspondant à une distance *d* selon la revendication 1 qui comprend la préparation d'une composition d'aluminosilicate cristalline ayant subi un échange avec l'ion hydrogène par le procédé selon la revendication 6, la mise en contact de cette composition avec un acide et l'isolement d'une composition d'aluminosilicate cristallin ayant subi en échange avec l'ion hydrogène présentant un rapport silice à alumine plus élevé et un diagramme de diffraction de rayons X de poudre correspondant à une distance *d* selon la revendication 1.

FIG. I

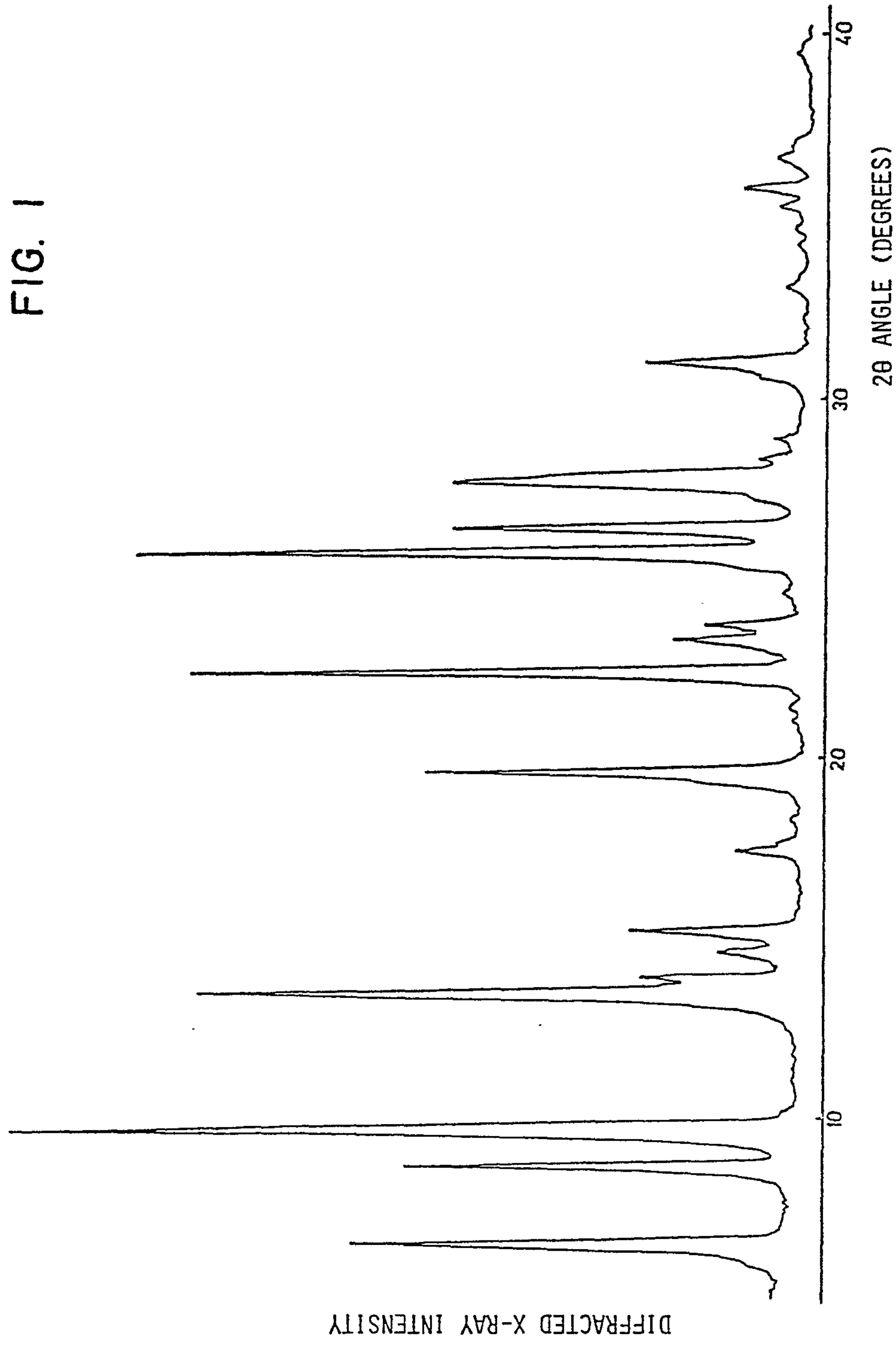

FIG. 2

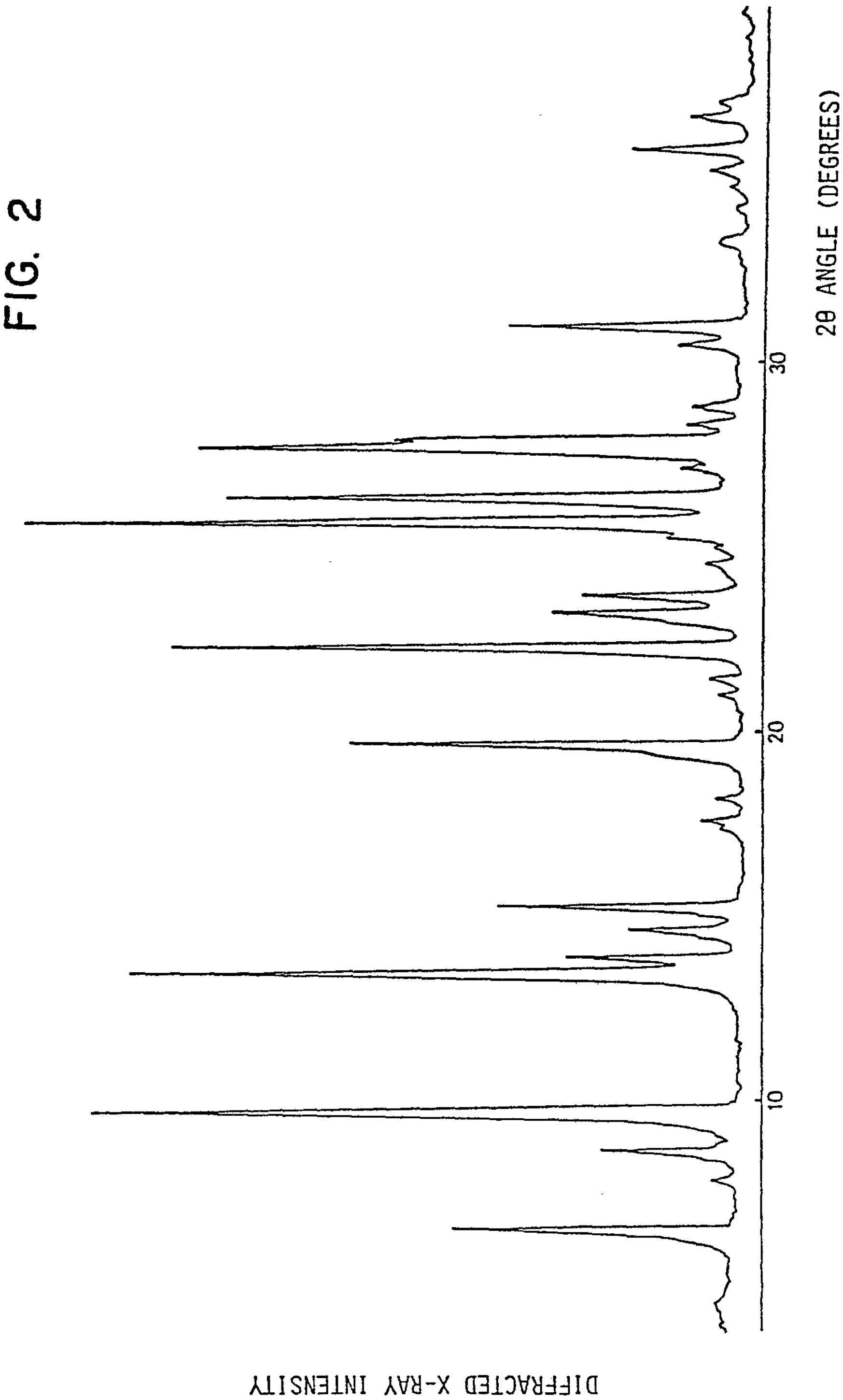

FIG. 3

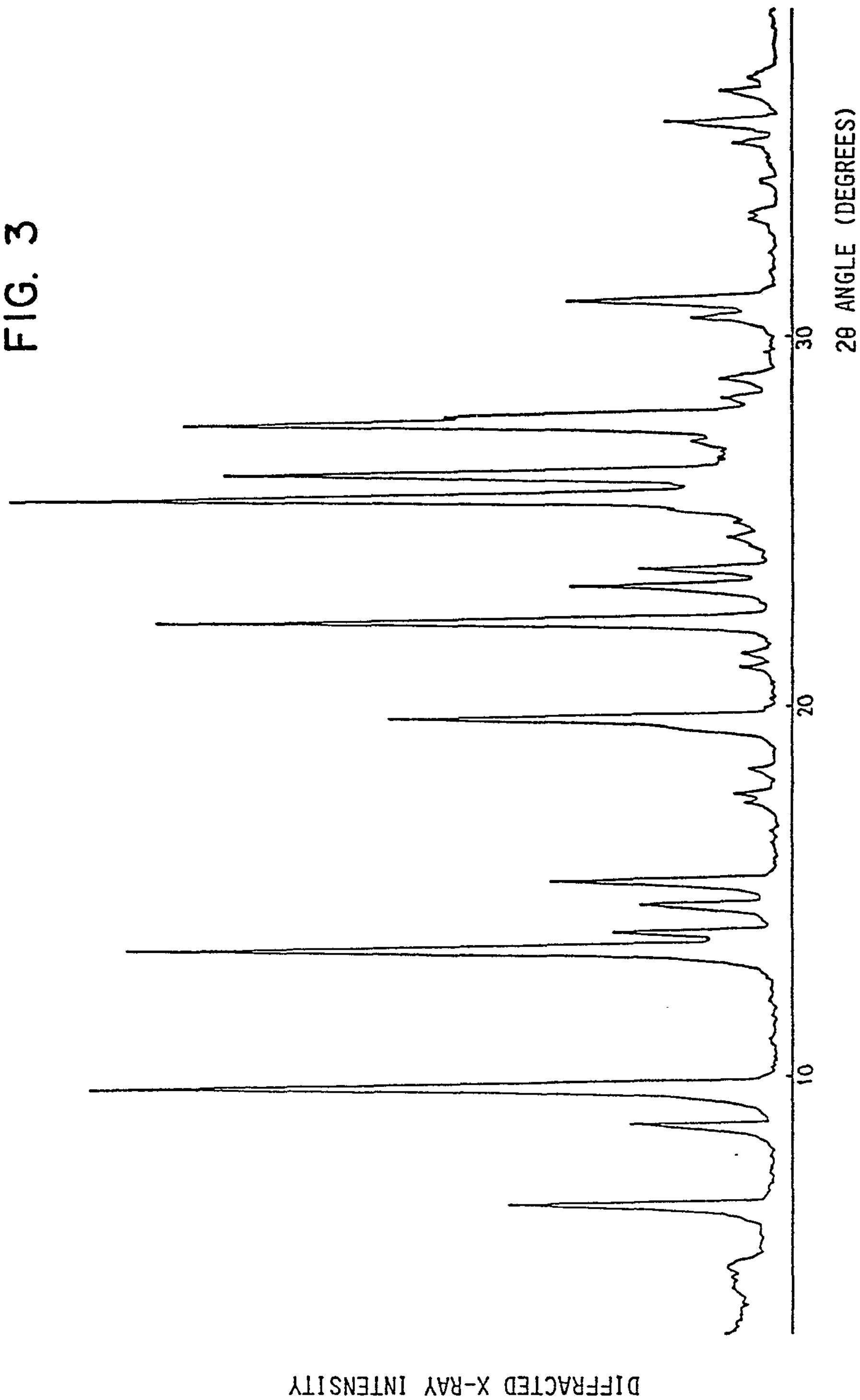

FIG. 4

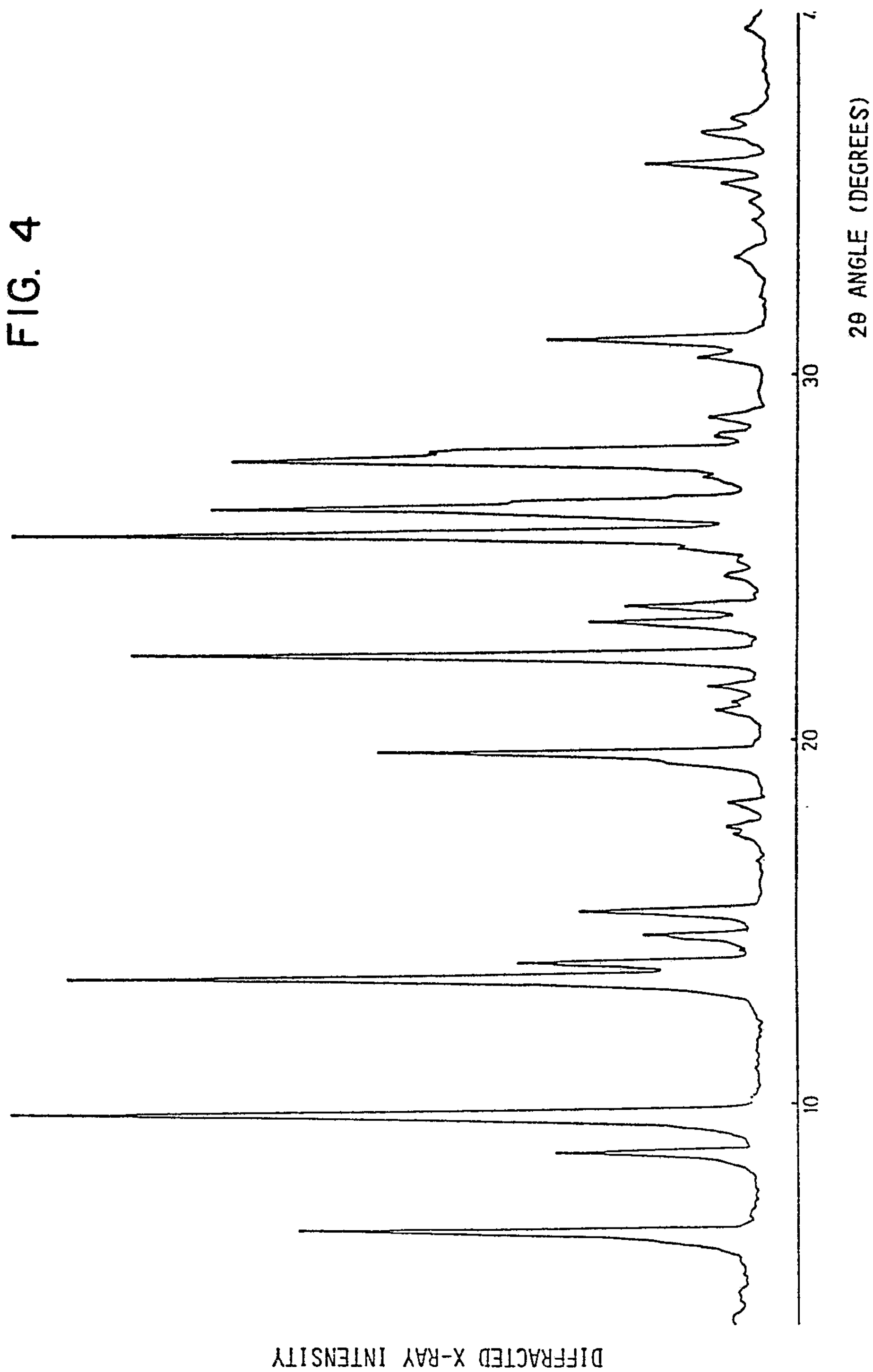

FIG. 5

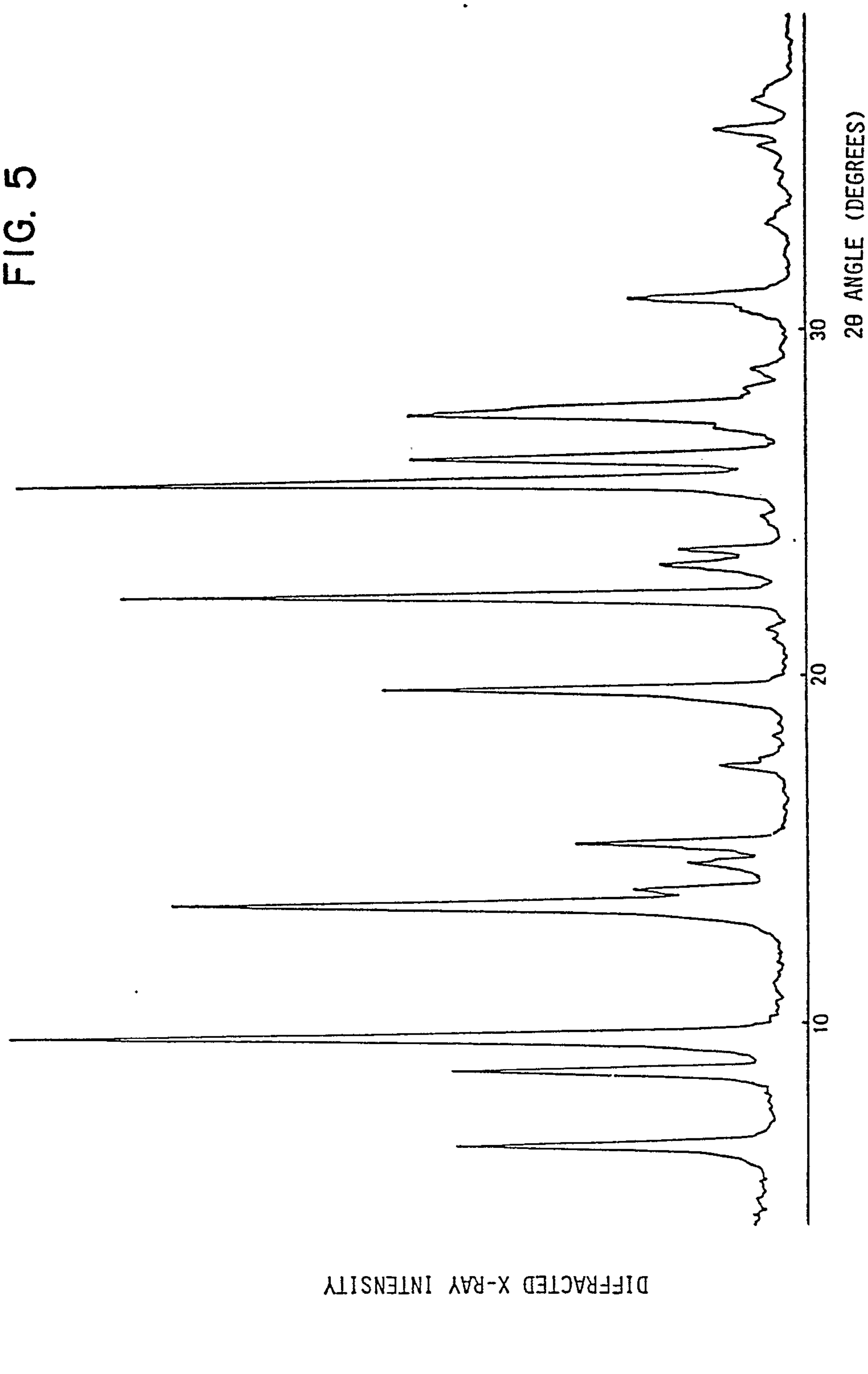

FIG. 6